(19) Europäisches Patentamt
European Patent Office
Office européen des brevets

(11) EP 0 658 096 B1

(12) **EUROPEAN PATENT SPECIFICATION**

(45) Date of publication and mention of the grant of the patent:
**03.11.1999 Bulletin 1999/44**

(21) Application number: **93921221.3**

(22) Date of filing: **27.08.1993**

(51) Int. Cl.[6]: **A61K 7/28**, A61K 38/44

(86) International application number:
**PCT/US93/08086**

(87) International publication number:
**WO 94/05252 (17.03.1994 Gazette 1994/07)**

(54) **STABILIZED CHEWABLE ANTIMICROBIAL ANIMAL FOODSTUFF**
STABILISIERTES, KAUBARES, ANTIMIKROBIELLES NAHRUNGSMITTEL FÜR TIERE
ALIMENT ANTIMICROBIEN STABILISE ET POUVANT ETRE MACHE, POUR ANIMAUX

(84) Designated Contracting States:
**DE ES FR GB IT NL**

(30) Priority: **27.08.1992 US 936929**

(43) Date of publication of application:
**21.06.1995 Bulletin 1995/25**

(73) Proprietor:
**MONTGOMERY, Robert E.**
**Monterrey, MA 01245 (US)**

(72) Inventor: **MONTGOMERY, Robert E.**
**Monterrey, MA 01245 (US)**

(74) Representative:
**Thomson, Paul Anthony et al**
**Potts, Kerr & Co.**
**15, Hamilton Square**
**Birkenhead Merseyside L41 6BR (GB)**

(56) References cited:
**US-A- 4 476 108**
**US-A- 4 537 764**
**US-A- 4 578 265**
**US-A- 5 011 679**
**US-A- 5 114 704**

- **RESEARCH DISCLOSURE., no.333, 1992, HAVANT GB page 2244 'Anti-microbial compositions'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 658 096 B1

## Description

This invention relates to a chewable object for animals which contains one or more enzymes and substrates for the purpose of generating antimicrobial compounds upon contact with an animal's saliva, in addition to containing catalase or other hydrogen peroxide- degrading enzymes in specified amounts to stabilize the activity of said chewable object during manufacture and storage. More specifically, chewable and consumable compositions are herein described which, upon being chewed, produce hydrogen peroxide or other antimicrobial agents.

## ART BACKGROUND:

A number of naturally occurring antimicrobial systems rely upon the ability of certain oxidizing agents to disrupt metabolic processes of bacteria, fungi and viruses. Compounds such as hypothiocyanite (OSCN-/HOSCN), hypochlorite (OCl-, HOCl), and hypoiodite (OI-, HOI) are known to inhibit glycolysis, penetrate prokaryotic cell walls, and generally disrupt a wide variety of processes crucial to the survival of lower organisms. These oxidizing agents are the result of the detoxification of hydrogen peroxide by mammalian peroxidase systems, such as those found in saliva, cervical fluid, lacrimal fluid, and leukocytes.

The antimicrobial effectiveness of the aforementioned oxidizing agents is well established in the literature and is known to begin to occur at concentrations of about 10 - 100 micromoles per liter. Hypothiocyanite ion (OSCN-/HOSCN) achieves antimicrobial effectiveness at around 100 micromoles per liter, while hypoiodite and hypochlorite ions ($OI^-$/HOI and $OCl^-$/HOCl, respectively) are effective inhibitors of microorganisms at concentrations as low as 10 to 50 micromoles per liter. The limiting factor in all of the mammalian antimicrobial peroxidase systems is usually the availability of hydrogen peroxide.

The numerous prior art attempts to activate or supplement natural antimicrobial peroxidase systems are generally limited to the oral care field. US 4,150,113 and US 4,178,362 (Hoogendorn, et al) describe dentifrices containing glucose oxidase in order to react with plaque and salivary glucose to produce low levels of hydrogen peroxide.

US 4,269,822, US 4,564,519 and US 4,578,265 (Pellico, et al.) further describe dentifrice compositions containing an oxidoreductase enzyme, together with its specific substrate, for the purpose of producing hydrogen peroxide or other antimicrobial oxidizing compounds, such as hypothiocyanite ion. In each of these prior art formulations, the oxidoreductase enzymes and substrates are in aqueous solution and homogeneously distributed throughout the entire mixture.

US 4,564,519 describes a chewable dentifrice, such as a chewing gum or lozenge, which contains a dual enzyme system for producing hypothiocyanite ions upon being chewed or otherwise activated by the moisture in saliva. All of the above compositions teach a manufacturing and compounding process whereby the enzymes, and, optionally, the substrates are distributed homogeneously throughout the entire composition.

In addition to US 4,564,519 above, other solid or chewable compositions capable of producing hydrogen peroxide or other oxidizing agents upon activation with moisture are taught in US 4,320,116, US 4,726,948, and US 4,929,466. These compositions are foodstuffs intended for consumption by livestock in order to limit the growth of harmful bacterial within the animal's gastrointestinal tract. The authors also teach a manufacturing and compounding process whereby the enzymes and substrates are distributed homogeneously throughout the composition.

It is well known to those skilled in the art that enzymes are reactive with their corresponding substrates when present together in aqueous solution. It is also well known by those skilled in the art that most enzymes are thermosensitive and frequently undergo a gradual, but irreversible inhibition when exposed to temperatures higher than 5 or 10 degrees Celsius for extended periods of time. This inhibition is particularly pronounced when thermolabile enzymes are exposed to such temperatures while in aqueous solution. It is also known that high concentrations of hydrogen peroxide are capable of inhibiting or destroying the activity of oxidoreductase and peroxidase enzymes.

Since domesticated animals, such as dogs are unable to practice any type of oral hygiene, they are more at risk for caries, gingivitis and periodontal disease than their humal counterparts. It would thus be advantageous to provide a chewable and consumable object for animals which contains or can produce agents effective in reducing or eliminating oral pathogens associated with such diseases.

It would also be advantageous to provide the chewable, consumable object as described above which contained an oxidoreductase enzyme, together with it's corresponding substrate, for the purpose of producing hydrogen peroxide upon being chewed.

It would also be advantageous to provide an enzymatically active chewable object as described above which was stabilized against premature production of hydrogen peroxide prior to use, irrespective of the amount of moisture contained within or on the surface of said object.

According to the present invention there is provided an antimicrobial animal chew comprising a carrier material, at least one oxidoreductase enzyme and at least one oxidoreductase substrate, said animal chew being made by the, process comprising:

a) providing a carrier;

b) applying either of said at least one enzyme or said at least one substrate to said carrier and substantially drying it thereon or therein;

c) applying the other of said substrate or said enzyme to said carrier and substantially drying it thereon; and

d) applying a catalase to said carrier; wherein said catalase is provided in an amount in the ratio range of 100 Titrimetric Units of oxidoreductase to 1.0 Baker Unit of catalase, to 1.0 Titrimetric Unit of oxidoreductase to 1.0 Baker Unit of catalase.

## SUMMARY OF THE INVENTION

The present invention provides a chewable and consumable composition which is capable of producing one or more antimicrobial agents upon contact with saliva, for the purpose of inhibiting, reducing, normalizing, or otherwise eliminating potentially harmful oral pathogens in the oral cavity of domesticated animals. More particularly, compositions are herein described which, upon being chewed, release an oxidoreductase enzyme, together with its corresponding substrate, into salivary solution in an animal's mouth, wherein the ensuing enzymatic reaction proceeds and produces hydrogen peroxide. The hydrogen peroxide thus produced is capable of activating the salivary peroxidase system already present in saliva, which in turn produces the potent antimicrobial species known as hypothiocyanite ion (OSCN-/HOSCN). The aforementioned compositions should be of a durable enough construction so that the residence, or chewing, time in an animal's mouth is of sufficient duration to allow the production or accumulation of a hydrogen peroxide concentration capable of raising salivary hypothiocyanite levels to at least about 100 micromoles per liter of salivary fluid.

The compositions of the present invention are supplied on an appropriate carrier which preferably is desirable for an animal to chew. The carrier, which is also called a chew herein, may be rawhide and rawhide-like compositions, biscuits, or dry animal food, sometimes referred to as kibble.

In the case of animal biscuits, kibble and other dry animal food, which obviously have a shorter duration of contact with the animal's mouth than a chew, such as rawhide and rawhide-like compositions, the effective compositions of the present invention may be provided with a proportionately higher level of oxidoreductase enzyme and substrate, such that hydrogen peroxide production will ensue and accumulate at a more rapid rate (commensurate with the lower expected oral residence time).

Once in salivary solution, the general reaction which ensues is as follows:

$$2\ \text{Substrate} + O_2 + H_2O \xrightarrow{\text{Oxidoreductase}} 2\ \text{Reduced substrate} + H_2O_2 + H_2O$$

The hydrogen peroxide produced in salivary solution as a result of the above reaction scheme is a further participant in the following sequence:

$$H_2O_2 + \text{thiocyanate ion } (SCN^-) \xrightarrow{\text{Salivary Peroxidase}}$$

$$\text{Hypothiocyanite ion } (OSCN^- / HOSCN) + H_2O$$

The hypothiocyanite ion ($OSCN^-$) exists in equilibrium with hypothiocyanous acid (HOSCN) while in solution, and the distribution of the two species is dependent upon pH. The pKa of hypothiocyanous acid (HOSCN) is 5.3. Since dissolution of enamel begins to occur at pH 5.5 and lower, and is thus undesirable, and since it is also known that the uncharged species hypothiocyanous acid (HOSCN) more readily penetrates the microbial cell wall, and is thus a more effective antimicrobial agent, the preferred pH of the present invention is in the range of 5.5 - 6.5 where the HOSCN species predominates.

The elevated levels of hypothiocyanite ($OSCN^-$/HOSCN) thus produced inhibits oral pathogens, as heretofore described.

It has also been discovered that the inclusion of catalase (E.C. 1.11.1.6) on the chew, in conjunction with the oxidoreductase enzyme of the present invention and its corresponding substrate, prevents the premature development

or accumulation of hydrogen peroxide during chew manufacture and/or storage. By catalyzing the breakdown of any hydrogen peroxide produced by the oxidoreductase enzyme and its corresponding substrate in the manufacturing process or during prolonged storage, due to the presence of residual moisture within and on the surface of the chew, catalase thus prevents any significant inhibition of the desired oxidoreductase activity until the intended time of use. At the point of use, however, the salivary peroxidase in saliva, having a much greater affinity for hydrogen peroxide than catalase, is seen to successfully utilize virtually all of the hydrogen peroxide produced for the oxidation of thiocyanate ion (SCN-), as described above.

## DETAILED DESCRIPTION OF THE INVENTION

In its simplest form, the antimicrobial animal chew of the present invention comprises a solid, chewable composition or material on or in which an oxidoreductase enzyme and its corresponding substrate, in addition to a specified amount of catalase or other hydrogen peroxide-degrading enzyme (with any attendant oxygen-accepting anion), have been applied or otherwise deposited. Prior to use, during manufacture and storage, any hydrogen peroxide that is produced as a result of the presence of residual moisture on the chew is destroyed by the catalase present. When in contact with an animal's saliva during the process of chewing, the oxidoreductase enzyme and its corresponding substrate are solubilized into aqueous solution, whence they react as previously described to produce hydrogen peroxide. The hydrogen peroxide produced is then preferentially utilized by salivary peroxidase (in relation to the catalase also present in salivary solution), to further oxidize thiocyanate ions ($SCN^-$) to the antimicrobially effective hypothiocyanite ions ($OSCN^-$/HOSCN).

The carrier is herein defined as the portion of the animal chew which is intended for the dog or other animal to chew on. The preferred carrier material of the present invention is rawhide. However, any material, composition, or construction which is safely consumable and durable enough to allow sufficient oral residence time for the oxidoreductase enzyme and its corresponding substrate to achieve solution in the salivary fluid, is contemplated. Other types of carrier material contemplated by the present invention include animal biscuits or crackers.

The solid, chewable carrier is preferably durable enough to prevent the animal from consuming it in less than about 1 to 5 minutes. This length of time is generally sufficient to allow the production of antimicrobial concentrations of hypothiocyanite ions, generally at a level of about 100 micromoles per liter of salivary fluid.

Suitable oxidoreductases include, but are not limited to, glucose oxidase, galactose oxidase, glycollate oxidase, lactate oxidase, L-gulunolactone oxidase, L-2-hydroxyacid oxidase, aldehyde oxidase, xanthine oxidase, D-aspartate oxidase, L-amino acid oxidase, D-amino acid oxidase, monoamine oxidase, pyridoxaminephosphate oxidase, diamine oxidase, and sulfite oxidase. The preferred oxidoreductase is glucose oxidase.

Suitable substrates are specific to the particular oxidoreductase chosen and are well known in the art. For instance, D-glucose is a specific substrate for glucose oxidase. Other suitable substrates include, but are not limited to D-glucose, D-galactose, L-sorbose, ethanol, tyramine, 1, 4-diaminobutane, 6-hydroxy-L-nicotine, 6-hydroxy-D-nicotine, 2-aminophenol, glycollate, L-lactate, 2-deoxy-D-Glucose, L-gulunolactone, L-galactonolactone, D-mannonolactone, L-2-hydroxyisocaproate, acetaldehyde, butyraldehyde, xanthine, D-aspartate, D-glutamate, L-amino acids and D-amino acids.

The antimicrobial animal chew of this invention comprises at least one of the above listed oxidoreductase enzymes together with its corresponding substrate, also listed above. For the purposes of this invention, a specific amount or activity of oxidoreductase enzyme shall be described in Titrimetric Units (TU), which is that amount of oxidoreductase enzyme capable of producing 1.1 micromoles of hydrogen peroxide per minute at 35° C., and optimal conditions of pH and substrate concentration for each particular oxidoreductase enzyme.

In general, the antimicrobial animal chews should contain at least about 1.0 TU of oxidoreductase enzyme per gram of carrier. A preferable level of oxidoreductase enzyme activity is from 5.0 to 50 TU per gram of carrier.

The corresponding substrate should be present in or on the antimicrobial animal chew at a level of at least 0.1 percent by weight of the total carrier weight (0.001 grams of substrate per gram of carrier). A preferable level of substrate is from 0.5 to 10 percent by weight of the total carrier weight. The most preferred range of substrate is from 0.5 to 5 percent by weight of the total carrier.

In order to stabilize the oxidoreductase activity of the animal chew against premature production of hydrogen peroxide due to residual moisture during the manufacturing process or storage, catalase may be advantageously included on or within the chew together with the aforementioned inventive components. Catalase enzyme activity, for the purpose of this invention and its attendant claims is measured in terms of Baker Units, One Baker Unit (BU) being equivalent to that amount of catalase capable of decomposing 264 mg of hydrogen peroxide in one hour under the conditions of the assay (25° C. and pH 7.0).

In general, the inventive animal chew should contain a minimum ratio of 1 BU of catalase to every 50 TU's of oxidoreductase enzyme, and more preferably a ratio of from about 1 BU of catalase : 20 TU's of oxidoreductase to about 1 BU of catalase : 1 TU of oxidoreductase enzyme. The most preferred ratio of catalase to oxidoreductase is from about

1 BU of catalase : 10 TU's of oxidoreductase to about 1 BU of catalase : 4 TU's of oxidoreductase.

Catalase derived from Asperiligus niger is preferred, especially when this organism is the source of the oxidoreductase enzyme, in order that the two enzymes can be cofermented to produce the correct ratio described above, and added to the chew as a single component. However, other sources of catalase, such as beef liver, are suitable for the practice of the present invention.

The antimicrobial animal chew may optionally contain or carry stabilizers, buffers, cofactors, and/or activators for the aforementioned oxidoreductase enzymes. Such optional components are intended to stabilize the activity and/or prevent denaturation of the oxidoreductase enzyme during the manufacture and storage of the inventive animal chew, and are well known in the art. Any such optional components must be biologically acceptable and suitable for ingestion by the animal. In addition, the suitability and function of an optional component as described herein is dependent upon the particular oxidoreductase enzyme present. Such optional components should not substantially contribute to the retention of additional water by the chew, so as to encourage and facilitate the interaction of oxidoreductase enzyme and its corresponding substrate.

In general, suitable optional components include polymeric stabilizers such as gelatin, albumin, and casein, in addition to stabilizing salts, such as sodium and potassium chloride. Suitable buffering components include potassium phosphate monobasic, potassium phosphate dibasic, sodium phosphate monobasic, and sodium phosphate dibasic, and may be included to adjust the animal's salivary pH during the chewing process to that which is optimal for the particular oxidoreductase enzyme selected. A suitable cofactor or activator (for glucose oxidase) is flavin adenine dinucleotide (FAD). Any optional component which can contribute to the preservation or potentiation of a particular oxidoreductase enzyme's activity within the parameters of its use on an antimicrobial animal chew is contemplated to be within the scope of this invention.

In addition to the above, the inventive animal chew may contain one or more peroxidase enzymes for the purpose of assuring the optimal utilization of the hydrogen peroxide produced by the oxidoreductase enzyme and its corresponding substrate. Suitable peroxidase enzymes include, but are not limited to, lactoperoxidase, chloroperoxidase, myeloperoxidase and salivary peroxidase. Any peroxidase capable of utilizing hydrogen peroxide to oxidize thiocyanate ions to form hypothiocyanite ions is contemplated. The preferred peroxidase is lactoperoxidase.

Peroxidase activity is measured, for the purpose of this invention, in ABTS Units. One ABTS Unit is the amount of peroxidase capable of oxidizing one micromole of ABTS (2,2'-azinobis(3-ethylbenzothiazoline-6-sulfonic acid)) per minute at 25° C., 0.1M phosphate buffer (pH 6.0), and initial hydrogen peroxide concentration of 1.0 mM.

In general, the antimicrobial animal chew may optionally contain or carry any level of peroxidase within economic practicality, however a preferred level is from 10.0 to 100 ABTS Units of peroxidase per gram of carrier.

The compositions of the present invention may also include a halide or pseudohalide ion source which is oxidizable by the specific peroxidase enzyme utilized. A thiocyanate ion source, such as potassium thiocyanate, sodium thiocyanate, ammonium thiocyanate, or other thiocyanate salts, may be included in order to optimize the final concentration of thiocyanate in the animal's saliva during the chewing cycle. The preferred thiocyanate ion source is potassium thiocyanate. Since salivary peroxidase is also capable of oxidizing iodide ions (I-) to antimicrobial hypoiodite ions (OI- / HOI), an iodide ion source such as potassium iodide may be included to enhance the chew's antimicrobial activity. Combinations of thiocyanate and iodide ions are also contemplated. Additionally, certain peroxidase enzymes, such as chloroperoxidase and myeloperoxidase, are also capable of utilizing hydrogen peroxide to oxidize chloride ions (Cl-) to hypochlorite ions (OCl- / HOCl); when these particular enzymes are included in the inventive composition, a chloride ion source such as sodium chloride is contemplated. The halide or pseudohalide ion sources are provided in the composition in the range of 0.1 micromoles of halide or pseudohalide per gram of carrier to 10 micromoles per gram of carrier. Preferably the concentration range of halide or pseudohalide in the carrier is 0.5 micromoles to 5 micromoles per gram of carrier, and most preferably, 1 to 2 micromoles of halide per gram of carrier.

Compositions of the present invention which contain both a peroxidase enzyme (other than catalase) and a halide or pseudohalide ion source are seen to obviate the need for catalase in order to be stabilized against premature production of hydrogen peroxide during manufacture or storage. For instance, a chew containing both lactoperoxidase and potassium thiocyanate, in addition to an oxidoreductase enzyme and its corresponding substrate, is stable against premature hydrogen peroxide production, whereas a chew containing merely the lactoperoxidase, in addition to an oxidoreductase enzyme and its corresponding substrate is not. In these instances, the peroxidase enzyme and a halide or pseudohalide ion source serves the same purpose as the catalase in the inventive compositions, that is, to scavenge and destroy any hydrogen peroxide produced in the course of manufacturing and storage.

Additional optional ingredients include flavorants and colorants, which are related to the organoleptic properties of the animal chew, and are well known in the art.

**EXAMPLE I**

Commercially available rectangular rawhide chews of a dimension approximately 2" wide and 4" in length were

individually weighed and then placed in a 15% (w/w) aqueous solution of dextrose (D-glucose) for a period of 120 minutes, then removed and placed in a constant temperature oven set at 45° C. to dry for 24 hours. After drying, the weight gain attributable to D-glucose deposition on the rawhide chew averaged 2.2 mg per gram of chew. The dry, D-glucose-impregnated chews were then contacted on one side only with a metered spray (0.125 ml) of one of the glucose oxidase solutions indicated below, and dried in a constant temperature oven at 45° C for 2 hours.

| Solution A | |
|---|---|
| Deionized water | 64.90 gms |
| Sodium chloride | 16.22 gms |
| Glucose oxidase solution (F100*, Genencor Intl) (100 TU glucose oxidase : 1 BU catalase ratio) | 18.88 gms |
| Solution A: 800 TU glucose oxidase/ml and <8BU catalase/ml | |

*F100: 5,000 TU glucose oxidase/ml and 100:1 glucose oxidase to catalase ratio (TU:BU)

| Solution B | |
|---|---|
| Deionized water | 37.33 gms |
| Sodium chloride | 9.33 gms |
| Glucose oxidase solution (Oxygo 1500, Genencor, Intl) | 53.34 gms |
| Oxygo 1500: 1,500 TU glucose oxidase/ml and 4:1 glucose oxidase to catalase ratio (TU:BU) | |
| Solution B: 800 TU glucose oxidase/ml and 200 BU catalase/ml | |

The final activity of the dried chews was calculated to be 10.0 TU per gram of chew, based on the volume of glucose oxidase solution applied prior to drying. Those chews treated with solution A were calculated to contain 0.1 BU of catalase per gram of chew, while those chews treated with Solution B were calculated to contain 2.5 BU of catalase per gram of chew.

The chews were assayed for hydrogen peroxide production, before and after extended storage as outlined below.

<u>In Vitro Hydrogen Peroxide Determination of Rawhide Animal Chew</u>

This procedure is based on the ability of a rawhide chew containing an oxidoreductase enzyme and its substrate (glucose oxidase and glucose, respectively) to produce hydrogen peroxide upon being vortexed with water from an artificial saliva composition. Since a distinction between the oxidoreductase activity maintained during manufacture and storage of a high catalase composition and a low catalase composition is sought in this assay, a coupled detection scheme was devised to accurately determine the amount of hydrogen peroxide produced prior to being scavenged by the catalase. A representative volume of artificial saliva was chosen based upon the assumption that a single chew, with an average weight of 10 grams would come in contact with approximately 15 milliliters or grams of water of an animal's saliva. Thus, 1.0 grams of rawhide chew is combined with 1.5 milliliter of artificial saliva (or any other multiple of this ratio) in the testing procedure

The detection of hydrogen peroxide in the "chew fluid" is based in part upon the procedure of Mansson-Rahemtulla, et al. (1986), *Arch. Oral Biol*. **31**, 661-668. , which utilizes an oxidizable chromogen to detect the presence of hypothiocyanite ions (OSCN- / HOSCN). The present procedure assumes that one mole of OSCN- / HOSCN will be produced for each mole of hydrogen peroxide generated by the rawhide / artificial saliva mixture.

Solutions

(a) Stock 5,5' - dithiobis (2-nitrobenzoic acid) (DTNB) was prepared by dissolving 58.2 mg of DTNB in 15.0 ml of 0.1 M $K_2HPO_4$ (pH 8.8) and made up to 100 ml with distilled water.

(b) The NBS assay buffer was prepared by adding 0.8 ml of 10 mM mercaptoethanol to 4.0 ml of stock DTNB in order to reduce DTNB to NBS. This solution was then made up to a final volume of 100 ml by addition of 0.1 M phosphate buffer (pH 5.6) to yield the working NBS buffer. This buffer should give an absorbance of 0.8 - 1.0 at 412 nm.

(c) Artificial saliva was prepared according to the following formula:

| | |
|---|---|
| 0.034 M phosphate buffer, pH 7.0 | 1000.0 ml |
| Sodium chloride | 1.4600 grams |
| Potassium thiocyanate | 0.1954 grams |
| Lactoperoxidase (500 ABTS Units/mg) | 5.0 mg |

Procedure

Note: An untreated rawhide chew was run as a control.

(a) The rawhide chew to be assayed was sliced into small squares, approximately 0.64 cm (1/4 inch) on a side, with large, durable scissors. 6.0 grams of rawhide chew squares were weighed and transferred to a 20 ml plastic scintillation vial with screw cap.

(b) 9.0 ml of Artificial Saliva (described above) was added to the vial, which was then vortexed for a period of three minutes, during which time 200 microliter samples of "chew fluid" were drawn at regular intervals (one minute, two minutes and three minutes).

(c) Each 200 microliter aliquot was immediately transferred to a tube in which 6.0 ml of the NBS solution had already been placed.

(d) The NBS/aliquot mixture was centrifuged for 1 minute at 15,000 x g.

(e) The change in absorbance of the sample was read at 412 nm against a reference prepared in the same manner, however using 200 microliters of "chew fluid" obtained by vortexing 6.0 grams of rawhide chew squares with 9.0 grams of distilled water.

Calculations

$$\text{Absorbance}_{412} = \frac{\text{Molar extinction coefficient (NBS)} \times [\text{OSCN-} / \text{HOSCN}]}{\text{Dilution factor}}$$

$$\text{Absorbance}_{412} = \frac{14{,}398\ M^{-1}\ cm^{-1} \times [\text{OSCN-} / \text{HOSCN}]}{32}$$

$$[\text{Hypothiocyanite ions in chew fluid}] = \text{Absorbance}_{412} \times 0.00111$$

$$[\text{Hydrogen peroxide in chew fluid}] = \text{Absorbance}_{412} \times 0.00111$$

An increase in absorbance of 1.00 at 412 nm is equivalent to a hydrogen peroxide (or hypothiocyanite ion) concentration of 1.11 millimoles per liter of "chew fluid".

Twelve chews, which were stored for 7 days at 25 deg. C. and 50% Relative Humidity following production were assayed according to the procedure outlined above. The average concentrations, in millimoles, of hydrogen peroxide generated by the inventive chews treated with either Solutions A and B above were as follows:

| | **60 second** | **120 second** | **180 second** |
|---|---|---|---|
| **Solution** | **[$H_2O_2$]** | **[$H_2O_2$]** | **[$H_2O_2$]** |
| A | 0.130 | 0.192 | 0.246 |
| B | 0.490 | 0.755 | 1.194 |

As can be seen in the above table, a marked difference in the ability of the inventive rawhide chew to produce hydrogen peroxide through the action of the oxidoreductase enzyme upon its substrate is observed between the chews treated with a high glucose oxidase to catalase unit ratio (Solution A) and those chews treated with a low glucose oxidase to catalase unit ratio (Solution B).

Twelve chews were also assayed which were stored for 30 days at 35 deg. C. and 50% Relative Humidity following production were assayed according to the procedure outlined above. The average concentrations, in millimoles, of hydrogen peroxide generated by the inventive chews treated with either Solutions A and B above were as follows:

| | **60 second** | **120 second** | **180 second** |
|---|---|---|---|
| **Solution** | **[$H_2O_2$]** | **[$H_2O_2$]** | **[$H_2O_2$]** |
| A | 0.035 | 0.040 | 0.033 |
| B | 0.395 | 0.699 | 1.028 |

The chews treated with Solution A have lost virtually all of their hydrogen peroxide producing capability through long term storage at elevated temperature and moderate humidity, whereas those chew treated with Solution B have retained their respective activity over the same time, under the same conditions of temperature and humidity, further indicating the importance of the presence of catalase in preserving the oxidoreductase enzyme activity in the inventive compositions.

EXAMPLE 2

Baked, uncoated dog biscuits were produced using the following dough composition:

| | |
|---|---|
| Water | 25 % |
| Wheat flour | 56 % |
| Soybean meal | 8 % |
| Meat and Bone meal | 7 % |
| Tallow (antioxidant-free) | 2 % |

(continued)

| | |
|---|---|
| Beef flavor | 2 % |

The above dough was shaped in the form of circular disks approximately 7.62 cm (3 inches) in diameter and 1.27 cm (1/2 inch) in thickness. The biscuit disks were baked at 400 - 450 degrees F. until a water content of 5 percent by weight was achieved (approximately 30 minutes).

After allowing the biscuits to cool to room temperature, the following flavor/substrate coating was applied at a rate of 4.9 percent by weight of biscuit:

| | |
|---|---|
| Water | 45Kg ( 100 lbs ) |
| Hydrolyzed vegetable protein | 5.9Kg ( 13 lbs ) |
| Beef/poultry digest | 5.9Kg ( 13 lbs ) |
| Dextrose | 11.25Kg( 25 lbs ) |
| Baker's yeast | 0.31Kg( 0.7 lbs ) |

Flavor/substrate coating was sprayed onto one side of the biscuit and allowed to dry for 2 hours at 105 degrees F.

The flavor/substrate-coated biscuits were then spray-coated with the following solution, at an application rate of 1.9 percent (w/w).

| | |
|---|---|
| Deionized water | 14. 85Kg ( 32.75lbs ) |
| Sodium chloride | 3.74Kg ( 8.25 lbs ) |
| Glucose oxidase solution (Oxygo 1500, Genencor, Intl) | 3.98Kg / ( 8.78 lbs ) |
| Oxygo 1500: 1,500 TU glucose oxidase/ml and 4:1 glucose oxidase to catalase ratio (TU:BU) | |

The oxidoreductase spray-coated biscuits were dried for 1 hour at 105 degrees F. and allowed to cool to room temperature. After 24 hours, these biscuits were tested for their ability to produce hydrogen peroxide by the method of Example 1. Results are shown in millimoles.

| 60 second | 120 second | 180 second |
|---|---|---|
| [$H_2O_2$] | [$H_2O_2$] | [$H_2O_2$] |
| 0.415 | 0.802 | 1.040 |

Thus, the dog biscuit of Example 2 demonstrates the ability to produce hydrogen peroxide at levels capable of activating the salivary peroxidase system while being chewed.

In addition to the rawhide and raw-hide like materials which may be used as a carrier, other carrier materials include baked animal chews or foodstuffs, such as cat kibble and dry dog food.

In addition to the process for manufacturing the antimicrobial animal chews of the present invention delineated above, other methods of applying an oxidoreductase enzyme and its corresponding substrate are contemplated. For example, in order to avoid the additional drying step at elevated temperature, a chew may be manufactured by contacting its surface with a granulated or otherwise solid mixture of oxidoreductase enzyme, substrate, and heretofore described optional components, in addition to granulating agents or carriers such as starch or proteinaceous material such as gelatin, casein and albumin. Such a granulated mixture adheres to a slightly moistened chew surface without dissolving, thus avoiding a premature reaction between the oxidoreductase enzyme and its corresponding substrate to

ensue.

The foregoing description of the invention is intended to be exemplary with respect to certain preferred embodiments and it will be understood that modifications and variations thereof obvious to those skilled in the art are to be included within the scope of this application and the appended claims.

## Claims

1. An antimicrobial animal chew comprising a carrier material, at least one oxidoreductase enzyme and at least one oxidoreductase substrate, said animal chew being made by the process comprising:

   a) providing a carrier;
   b) applying either of said at least one enzyme or said at least one substrate to said carrier and substantially drying it thereon or therein;
   c) applying the other of said substrate or said enzyme to said carrier and substantially drying it thereon; and
   d) applying a catalase to said carrier; wherein said catalase is provided in an amount in the ratio range of 100 Titrimetric Units of oxidoreductase to 1.0 Baker Unit of catalase, to 1.0 Titrimetric Unit of oxidoreductase to 1.0 Baker Unit of catalase.

2. An animal chew as claimed in claim 1, wherein the oxidoreductase enzyme of step b) and the catalase of step d) are applied together to said carrier in a single step.

3. An animal chew as claimed in claim 1, wherein the oxidoreductase enzyme of step b) and the catalase of step d) are applied separately to said carrier.

4. An animal chew as claimed in claim 1, 2 or 3, wherein said oxidoreductase enzyme is selected from glucose oxidase, galactose oxidase, glycollate oxidase, lactate oxidase, L-gulunolactone oxidase, L-2-oxidase, L-amino acid oxidase, D-amino acid oxidase, monoamine oxidase, pyridoxaminephosphate oxidase, diamine oxidase, and sulfite oxidase.

5. An animal chew as claimed in claim 4, wherein said oxidoreductase is glucose oxidase.

6. An animal chew as claimed in any preceding claim, wherein said substrates are specific to the particular oxidoreductase and are selected from D-glucose, D-galactose, L-sorbose, ethanol, tyramine, 1, 4-diaminobutane, 6-hydroxy-L-nicotine, 6-hydroxy-D-nicotine, 2-aminophenol, glycollate, L-lactate, 2-deoxy-D-Glucose, L-gulunolactone, L-galactonolactone, D-mannonolactone, L-2--hydroxyisocaproate, acetaldehyde, butyraldehyde, xanthine, D-aspartate, D-glutamate, L-amino acids and D-amino acids.

7. An animal chew as claimed in claim 6, wherein said oxidoreductase is glucose oxidase and said substrate is D-glucose.

8. An animal chew as claimed in any preceding claim wherein said oxidoreductase substrate is applied to said carrier and substantially dried thereon, and thereafter, a mixture of said oxidoreductase enzyme, a catalase, a second peroxidase and a halide or pseudohalide ion source capable of serving as an oxygen acceptor for said second peroxidase is applied to said carrier and substantially dried thereon.

9. An animal chew as claimed in claim 8 further comprising providing to said carrier:

   a peroxidase enzyme selected from the group consisting of lactoperoxidase, salivary peroxidase, chloroperoxidase, and myeloperoxidase, and
   a source of halide or pseudohalide ions capable of serving as an oxygen acceptor for said peroxidase enzyme, wherein the ions are selected from potassium thiocyanate, sodium thiocyanate, ammonium thiocyanate, other thiocyanate salts, potassium iodide, other iodide salts, sodium chloride, and other chloride salts.

10. An amimal chew as claimed in claim 9, wherein said halide or pseudohalide ions are present in the carrier in a range of 0.1 micromoles per gram of carrier to 10 micromoles per gram.

11. An animal chew as claimed in claim 10, wherein said halide or pseudohalide ions are present in said carrier in a range of 0.5 to 5 micromoles per gram of carrier.

12. An animal chew as claimed in claim 11, where said halide or pseudohalide ions are present in said carrier in the range of 1 to 2 micromoles per gram of carrier.

13. An animal chew as claimed in any preceding claim, wherein the catalase is derived from A. niger fermentation.

14. An animal chew as claimed in any preceding claim, wherein the carrier is selected from rawhide and rawhide-like materials, animal biscuits and dry animal food.

15. An animal chew as claimed in claim 14, wherein the carrier is rawhide.

16. An animal chew as claimed in claim 14, wherein the carrier is a dog biscuit.

17. An animal chew as claimed in claim 14, wherein the carrier is a dry animal food.

18. An animal chew as claimed in any preceding claim, wherein said oxidoreductase substrate is absorbed into said animal chew in liquid form, and then dried therein, and said oxidoreductase enzyme and a catalase are coated on the outside of said animal chew and then dried therein.

19. An animal chew as claimed in any preceding claim, wherein said oxidoreductase enzyme is present at a level of at least about 1.0 TU of oxidoreductase enzyme per gram of carrier material.

20. An animal chew as claimed in claim 19, wherein the level of oxidoreductase enzyme activity is in the range of 5.0 to 50 TU per gram of carrier.

21. An animal chew as claimed in any preceding claim, wherein said substrate is present in or on the antimicrobial animal chew at a level of at least 0.1 percent by weight of the carrier material.

22. An animal chew as claimed in claim 21, wherein said substrate level is in the range from 0.5 to 10 percent by weight of the carrier material.

23. An animal chew as claimed in claim 9, wherein said peroxidase is present in the range of from 10.0 to 100 ABTS Units of peroxidase per gram of carrier.

24. An animal chew as claimed in any preceding claim, wherein the step of drying said enzyme on said chew comprises drying said chew at 45°C. for 24 hours.

**Patentansprüche**

1. Antimikrobielles Kaumittel für Tiere, das ein Trägermaterial, wenigstens ein Oxidoreduktase-Enzym und wenigstens ein Oxidoreduktase-Substrat umfaßt, wobei das Kaumittel für Tiere durch ein folgende Schritte umfassendes Verfahren hergestellt wird:

   a) Bereitstellen eines Trägers;
   b) Auftragen des entweder wenigstens einen Enzyms oder des wenigstens einen Substrats auf den Träger und im wesentlichen Trocknen von diesen Stoffen darauf oder darin;
   c) Auftragen des jeweils anderen Stoffes von dem Substrat und dem Enzym auf den Träger und im wesentlichen Trocknen von diesem Stoff darauf;
   d) Auftragen einer Katalase auf den Träger;

   wobei die Katalase in einer Menge im Verhältnisbereich von 100 Titrimetrischen Units von Oxidoreduktase zu 1,0 Baker Units von Katalase bis zu 1,0 Titrimetrischen Units von Oxidoreduktase zu 1,0 Baker Units von Katalase eingesetzt wird.

2. Kaumittel für Tiere nach Anspruch 1, bei dem das Oxidoreduktase-Enzym von Schritt b) und die Katalase von Schritt d) zusammen in einem einzigen Schritt auf den Träger aufgetragen werden.

3. Kaumittel für Tiere nach Anspruch 1, bei dem das Oxidoreduktase-Enzym von Schritt b) und die Katalase von Schritt d) getrennt auf den Träger aufgetragen werden.

4. Kaumittel für Tiere nach Anspruch 1,2 oder 3, bei dem das Oxidoreduktase-Enzym aus Glukose-, Galaktose-, Glykolat-, Laktat-, L-Gulonolakton-, L-2-Hydroxysäure-, L-Aminosäure-, D-Aminosäure-, Monoamin-, Pyridoxaminphosphat-, Diamin- und Sulfit-Oxidase ausgewählt ist.

5. Kaumittel für Tiere nach Anspruch 4, bei dem das Oxidoreduktase-Enzym Glukose-Oxidase ist.

6. Kaumittel für Tiere nach einem der vorstehenden Ansprüche, bei dem die Substrate spezifisch für die einzelnen Oxidoreduktasen sind und aus D-Glukose, D-Galaktose, L-Sorbose, Ethanol, Tyramin, 1,4- Diaminobutan, 6-Hydroxy-L-nikotin, 6-Hydroxy-D-nikotin, 2-Aminophenol, Glykolat, L-Laktat, 2-Desoxy-D-Glukose, L-Gulonolakton, L-Galaktonolakton, D-Mannonolacton, L-2-Hydroxyisocapronat, Acetaldehyd, Butyraldehyd, Xanthin, D-Aspartat, D-Glutamat, L-Aminosäuren und D-Aminosäuren ausgewählt sind.

7. Kaumittel für Tiere nach Anspruch 6, bei dem die Oxidoreduktase Glukose-Oxidase und das Substrat D-Glukose sind.

8. Kaumittel für Tiere nach einem der vorstehenden Ansprüche, bei dem das Oxidoreduktase-Substrat auf den Träger aufgetragen und darauf im wesentlichen getrocknet wird, und danach ein Gemisch des Oxidoreduktase-Enzyms, einer Katalase, einer zweiten Peroxidase und einer Halogenid- oder Pseudohalogenid-Ionenquelle, die als Sauerstoffakzeptor für die zweite Peroxidase geeignet ist, auf den Träger aufgetragen und darauf im wesentlichen getrocknet wird.

9. Kaumittel für Tiere nach Anspruch 8, das ferner folgende Stoffe zum Auftragen auf den Träger umfaßt:

- ein Peroxidase-Enzym, ausgewählt aus der Gruppe, bestehend aus Lakto-, Salivary-, Chlor- und Myelo-Peroxidase, und
- eine Quelle von Halogenid- oder Pseudohalogenid-Ionen, die als Sauerstoffakzeptor für das Peroxidase-Enzym geeignet ist,

wobei die Ionen aus Kaliumthiocyanat, Natriumthiocyanat, Ammoniumthiocyanat, anderen Thiocyanatsalzen, Kaliumiodid, anderen Iodidsalzen, Natriumchlorid und anderen Chloridsalzen ausgewählt sind.

10. Kaumittel für Tiere nach Anspruch 9, bei dem die Halogenid- oder Pseudohalogenid-Ionen in dem Träger in einem Bereich von 0,1 Mikromol pro Gramm Träger bis 10 Mikromol pro Gramm vorliegen.

11. Kaumittel für Tiere nach Anspruch 10, bei dem die Halogenid- oder Pseudohalogenid-Ionen in dem Träger in einem Bereich von 0,5 bis 5 Mikromol pro Gramm Träger vorliegen.

12. Kaumittel für Tiere nach Anspruch 11, bei dem die Halogenid- oder Pseudohalogenid-Ionen in dem Träger in einem Bereich von 1 bis 2 Mikromol pro Gramm Träger vorliegen.

13. Kaumittel für Tiere nach einem der vorstehenden Ansprüche, bei dem die Katalase aus A.niger Fermentation gewonnen wird.

14. Kaumittel für Tiere nach einem der vorstehenden Ansprüche, bei dem der Träger aus ungegerbter Haut, einem ungegerbter Haut ähnlichen Material, Keksen für Tiere und Trockenfutter für Tiere ausgewählt ist.

15. Kaumittel für Tiere nach Anspruch 14, bei dem der Träger ungegerbte Haut ist.

16. Kaumittel für Tiere nach Anspruch 14, bei dem der Träger ein Keks für Hunde ist.

17. Kaumittel für Tiere nach Anspruch 14, bei dem der Träger Trockenfutter für Tiere ist.

18. Kaumittel für Tiere nach einem der vorstehenden Ansprüche, bei dem das Oxidoreduktase-Substrat von dem Kaumittel für Tiere in flüssiger Form absorbiert und dann darin getrocknet wird, und die Außenseite des Kaumittels für Tiere mit dem Oxidoreduktase-Enzym und einer Katalase überzogen und dann darin getrocknet wird.

19. Kaumittel für Tiere nach einem der vorstehenden Ansprüche, bei dem das Niveau der Oxidoreduktase-Enzym-Aktivität bei wenigstens etwa 1,0 TU des Oxidoreduktase-Enzyms pro Gramm Trägermaterial liegt.

20. Kaumittel für Tiere nach Anspruch 19, bei dem das Niveau der Oxidoreduktase-Enzym-Aktivität im Bereich von 5,0 bis 50 TU pro Gramm Träger liegt.

21. Kaumittel für Tiere nach einem der vorstehenden Ansprüche, bei dem das Substrat in oder auf dem antimikrobiellen Kaumittel für Tiere in einer Menge von wenigstens 0,1 Gewichtsprozent des Trägermaterials vorliegt.

22. Kaumittel für Tiere nach Anspruch 21, bei dem das Substrat in einer Menge im Bereich von 0,5 bis 10 Gewichtsprozent des Trägermaterials vorliegt.

23. Kaumittel für Tiere nach Anspruch 9, bei dem die Peroxidase im Bereich von 10,0 bis 100 ABTS-Units der Peroxidase pro Gramm Träger vorliegt.

24. Kaumittel für Tiere nach einem der vorstehenden Ansprüche, bei dem der Trocknungsschritt für das Enzym auf dem Kaumittel eine 24 Stunden lange Trocknung des Kaumittels bei 45°C umfaßt.

**Revendications**

1. Produit à mâcher antimicrobien pour animaux, comprenant un matériau support, au moins une enzyme oxydoréductase et au moins un substrat pour oxydoréductase, ledit produit à mâcher pour animaux étant fabriqué par le procédé qui consiste :

   a) à fournir un support ;
   b) à appliquer au moins ladite enzyme ou au moins ledit substrat sur ledit support, et à l'y soumettre à un séchage presque complet ;
   c) à appliquer l'autre dudit substrat ou de ladite enzyme sur ledit support, et à l'y soumettre à un séchage presque complet ; et
   d) à appliquer une catalase sur ledit support ;

   où ladite catalase est fournie en une quantité faisant un rapport compris entre 100 unités titrimétriques d'oxydoréductase à 1,0 unité Baker de catalase, et 1,0 unité titrimétrique d'oxydoréductase à 1,0 unité Baker de catalase.

2. Produit à mâcher pour animaux selon la revendication 1, dans lequel l'enzyme oxydoréductase de l'étape b) et la catalase de l'étape d) sont appliquées ensemble audit support en une étape unique.

3. Produit à mâcher pour animaux selon la revendication 1, dans lequel l'enzyme oxydoréductase de l'étape b) et la catalase de l'étape d) sont appliquées séparément audit support.

4. Produit à mâcher pour animaux selon la revendication 1, 2 ou 3, dans lequel ladite enzyme oxydoréductase est choisie parmi la glucose-oxydase, la galactose-oxydase, la glycolate-oxydase, la lactate-oxydase, la L-gulunolactone-oxydase, la L-2-oxydase, la (acide L-aminé)-oxydase, la (acide D-aminé)-oxydase, la monoamineoxydase, la pyridoxaminephosphate-oxydase, la diamine oxydase et la sulfite oxydase.

5. Produit à mâcher pour animaux selon la revendication 4, dans lequel ladite oxydoréductase est la glucose oxydase.

6. Produit à mâcher pour animaux selon l'une quelconque des revendications précédentes, dans lequel lesdits substrats sont spécifiques de l'oxydoréductase particulière et sont choisis parmi le D-glucose, le D-galactose, le L-sorbose, l'éthanol, la tyramine, le 1,4-diaminobutane, la 6-hydroxy-L-nicotine, la 6-hydroxy-D-nicotine, le 2-aminophénol, les glycolates, les L-lactates, le 2-désoxy-D-glucose, la L-gulunolactone, la L-galactonolactone, la D-mannonolactone, les L-2-hydroxy-isocaproates, l'acétaldéhyde, le butyraldéhyde, la xanthine, les D-aspartates, les D-glutamates, les acides L-aminés et les acides D-aminés.

7. Produit à mâcher pour animaux selon la revendication 6, dans lequel ladite oxydoréductase est la glucoseoxydase, et ledit substrat est le D-glucose.

8. Produit à mâcher pour animaux selon l'une quelconqué des revendications précédentes, dans lequel ledit substrat pour oxydoréductase est appliqué sur ledit support et y est soumis à un séchage presque complet, puis on applique audit support un mélange de ladite enzyme oxydoréductase, d'une catalase, d'une deuxième peroxydase et d'une source d'un ion halogénure ou pseudo-halogénure capable de servir d'accepteur d'oxygène pour ladite

deuxième peroxydase, et on l'y soumet à un séchage presque complet.

9. Produit à mâcher pour animaux selon la revendication 8, caractérisé en outre en ce qu'il consiste à placer sur ledit support :

   une enzyme peroxydase choisie dans le groupe comprenant la lactoperoxydase, la peroxydase salivaire, la chloroperoxydase et la myéloperoxydase, et
   une source d'ions halogénure ou pseudohalogénure capable de servir d'accepteur d'oxygène pour ladite enzyme peroxydase, où les ions sont choisis parmi le thiocyanate de potassium, le thiocyanate de sodium, le thiocyanate d'ammonium, d'autres sels thiocyanates, l'iodure de potassium, d'autres sels iodures, le chlorure de sodium et d'autres sels chlorures.

10. Produit à mâcher pour animaux selon la revendication 9, dans lequel lesdits ions halogénure ou pseudohalogénure sont présents dans le support en une quantité de 0,1 à 10 micromoles par gramme de support.

11. Produit à mâcher pour animaux selon la revendication 10, dans lequel lesdits ions halogénure ou pseudohalogénure sont présents dans ledit support en une quantité de 0,5 à 5 micromoles par gramme de support.

12. Produit à mâcher pour animaux selon la revendication 11, dans lequel lesdits ions halogénure ou pseudohalogénure sont présents dans ledit support en une quantité de 1 à 2 micromoles par gramme de support.

13. Produit à mâcher pour animaux selon l'une quelconque des revendications précédentes, dans lequel la catalase dérive de la fermentation de A. niger.

14. Produit à mâcher pour animaux selon l'une quelconque des revendications précédentes, dans lequel le support est choisi parmi le cuir parcheminé et les matériaux de type cuir parcheminé, les biscuits pour animaux et les aliments secs pour animaux.

15. Produit à mâcher pour animaux selon la revendication 14, dans lequel le support est un cuir parcheminé.

16. Produit à mâcher pour animaux selon la revendication 14, dans lequel le support est un biscuit pour chien.

17. Produit à mâcher pour animaux selon la revendication 14, dans lequel le support est un aliment sec pour animaux.

18. Produit à mâcher pour animaux selon l'une quelconque des revendications précédentes, dans lequel ledit substrat pour oxydoréductase est absorbé par ledit produit à mâcher pour animaux sous forme liquide, puis y est séché, et ladite enzyme oxydoréductase et une catalase sont appliquées sur l'extérieur dudit produit à mâcher pour animaux, puis y sont séchées.

19. Produit à mâcher pour animaux selon l'une quelconque des revendications précédentes, dans lequel ladite enzyme oxydoréductase est présente en une quantité d'au moins environ 1,0 UT d'enzyme oxydoréductase par gramme de matériau support.

20. Produit à mâcher pour animaux selon la revendication 19, dans lequel le niveau d'activité de l'enzyme oxydoréductase est compris entre 5,0 et 50 UT par gramme de support.

21. Produit à mâcher pour animaux selon l'une quelconque des revendications précédentes, dans lequel ledit substrat est présent dans ou sur le produit à mâcher antimicrobien pour animaux en une quantité d'au moins 0,1 % en poids par rapport au matériau support.

22. Produit à mâcher pour animaux selon la revendication 21, dans lequel ladite quantité de substrat est comprise entre 0,5 et 10 % en poids par rapport au matériau support.

23. Produit à mâcher pour animaux selon la revendication 9, dans lequel ladite peroxydase est présente en une quantité de 10,0 à 100 unités ABTS de peroxydase par gramme de support.

24. Produit à mâcher pour animaux selon l'une quelconque des revendications précédentes, dans lequel l'étape de séchage de ladite enzyme sur ledit produit à mâcher consiste à sécher ledit produit à mâcher à 45°C pendant 24

heures.